Europäisches Patentamt

European Patent Office

Office européen des brevets

(11) Veröffentlichungsnummer: **0 129 696**

**A1**

(12) **EUROPÄISCHE PATENTANMELDUNG**

(21) Anmeldenummer: **84105599.9**

(22) Anmeldetag: **17.05.84**

(51) Int. Cl.⁴: **G 01 N 33/50**
**G 01 N 33/92**

(30) Priorität: **26.05.83 DE 3319066**

(43) Veröffentlichungstag der Anmeldung:
**02.01.85 Patentblatt 85/1**

(84) Benannte Vertragsstaaten:
**IT**

(71) Anmelder: **Boehringer Mannheim GmbH**
**Sandhoferstrasse 116**
**D-6800 Mannheim 31(DE)**

(72) Erfinder: **Heuck, Claus-Christian**
**Birkenweg 20**
**D-6901 Wilhelmsfeld(DE)**

(54) Verfahren und Reagenz zum direkten Nachweis eines Analyts nach spezifischer Entfernung von Colloidpartikeln in wässrigen Lösungen biologischer Herkunft.

(57) Zur direkten Bestimmung eines Analyts wie z.B. Cholesterin aus einer bestimmten Fraktion in Körperflüssigkeiten, entfernt man andere den Analyt ebenfalls enthaltende Fraktionen durch eine Immunreaktion und bestimmt anschließend direkt den Analyt.

EP 0 129 696 A1

Croydon Printing Company Ltd.

1

## Beschreibung

Die Erfindung betrifft ein Verfahren zum Zwecke des Nachweises eines Analyts,der in verschiedenen Fraktionen an Biopolymere gebunden in einer Flüssigkeit biologischer Herkunft vorliegt und nur in einer dieser Fraktionen qualitativ und/oder quantitativ gemessen werden soll.Das Prinzip des Verfahrens wird am Beispiel der Untersuchung von Lipiden in Lipoproteinen des menschlichen Serums erläutert.

Die wasserunlöslichen Fette, Triglyceride, Cholesterin und Phospholipide, werden im wässrigen Milieu des menschlichen Plasma in mizellähnlichen Bestandteilen, den Serumlipoproteinen, transportiert. Es können verschiedene Lipoproteinklassen im menschlichen Nüchternserum nachgwiesen werden. Sie alle enthalten Triglycerid, Cholesterin, Phospholipid und bestimmte Proteine, sogenannte Apolipoproteine. Die drei im menschlichen Serum nachweisbaren, großen Lipoproteinklassen unterscheiden sich in ihrer spezifischen Dichte. Sie werden infolge dessen als Very Low Density Lipoproteine (VLDL), Low Density Lipoproteine (LDL) und High Density Lipoproteine (HDL) bezeichnet. Die Apolipoproteine werden ebenfalls in verschiedene Klassen unterteilt, die mit einem Buchstaben gekennzeichnet sind (z.B. Apolipoprotein A (Apo-A), Apolipoprotein B (Apo-B), Apolipoprotein C (Apo-C), Apolipoprotein D (Apo-D) und Apolipoprotein E (Apo-E). Kennzeichnenderweise sind die Apolipoproteinklassen meist nicht auf eine sondern mehrere Lipoproteinklassen verteilt. So enthalten die VLDL Apo-A, Apo B, Apo-C und Apo-E, die HDL enthalten Apo-A,
Apo-C und Apo-E. Hingegen enthalten die LDL nahezu
ausschließlich Apo-B.

Die pathognomonische Bedeutung der einzelnen Lipoproteinklassen ist für die Ablagerung von Fetten,
insbesondere von Cholesterin, in den Gefäßwänden
unterschiedlich. Die LDL bestehen zum größten Teil
aus Cholesterin. Ihr Beitrag zur Ablagerung von
Cholesterin in den Gefäßen - ein Prozeß, der als
Atherogenese bezeichnet wird - ist nachgewiesenermaßen am größten. Die HDL sind Phospholipid-reich.
Sie vermögen Cholesterin aus den Zellen der Gefäße
aufzunehmen und abzutransprotieren. Ihnen wird daher
eine anti-atherogene Wirkung nachgesagt.

In klinisch-chemischen Untersuchungen wurde herausgefunden, daß Personen mit erhöhten LDL-Konzentrationen
im Plasma oder Serum ein höheres Risiko haben, an Herz-
Kreislauferkrankungen zu leiden, während Personen mit
erhöhten HDL-Konzentrationen deutlich weniger anfällig
sind. Es hat sich daher für die labormedizinische Diagnostik als vorteilhaft erwiesen, nicht nur die Bestimmung von Gesamtcholesterin im Serum sondern auch
die Bestimmung von Cholesterin in der LDL-Fraktion
bzw. in der HDL-Fraktion zur prognostischen Beurteilung
des Ausmaßes der Gefährdung einer Person in Betracht
zu ziehen. Insbesondere der Bestimmung von Cholesterin
in den LDL wird eine herausragende Bedeutung beigemessen.

Die Bestimmung von LDL-Cholesterin aus Plasma oder
Serum wird herkömmlicherweise mit Hilfe kombinierter
Verfahren, z.B. der Ultrazentrifugation und Polyanionenpräzipitation in Speziallaboratorien (Lipid Research Clinics Program, DHEW Publication No (NIH)
75-628, Bethesda, Md. (1974)) oder aus den Konzentrationen von Gesamtcholesterin, Gesamttriglycerid und
HDL-Cholesterin im Serum durch Berechnung mit Hilfe
der Formel nach Friedewald          (Clin. Chem.
18, 499 (1972) durchgeführt. Der Nachteil dieser Methoden ergibt sich aus der indirekten Bestimmung der
Meßgröße, da hierbei die Fehler aller Messungen in
die Berechnung des Wertes für LDL-Cholesterin einfließen.

Überraschenderweise wurde festgestellt, daß die direkte Bestimmung von Lipiden, z.B. Cholesterin, in
einer bestimmten Lipoproteinfraktion, in hervorragender Weise ausgeführt werden kann, wenn aus der
zu untersuchenden Probe die Lipoproteinpartikel, die
zwar auch Lipide enthalten, jedoch nicht der zu untersuchenden Lipoproteinfraktion zugeordnet sind, durch
eine spezifische Immunreaktion entfernt werden. Das
Verfahren setzt voraus, daß das Reagenz immunchemische Eigenschaften hat, die ausschließlich gegen
die zu entfernenden Lipoproteine gerichtet sind.
Beispielsweise reagiert ein Reagenz mit immunchemischen Eigenschaften gegen Apo A und Apo C mit VLDL-
und HDL-Partikeln, die beide Apo A und Apo C enthalten.
Es reagiert hingegen nicht mit LDL-Partikeln, die diese
Apolipoproteine nicht enthalten. Mit Hilfe einer spezifischen immunchemischen Reaktion können die in einer
Probe vorhandenen verschiedenen Lipoproteinklassen
voneinander getrennt werden. Der Gehalt an Lipiden in
der Restlösung entspricht somit dem Lipidgehalt der
nicht umgesetzten Lipoproteinfraktionen unter der Voraussetzung, daß das Immunreagenz Lipide gleicher Art
in Mengen enthält, die die Messung nicht beeinträchtigen. Aus der Restlösung kann daher der Lipidhehalt -

4

gegebenenfalls unter Berücksichtigung eines Leerwertes
- direkt gemessen werden. Da das Plasma aller Tierspezies, die zur Antikörpersynthese immunisiert werden,
physiologischerweise Lipide enthält, die den quantitativen Nachweis eines entsprechenden Analyts in einem
Humanserum beeinträchtigen würden, müssen diese zuvor
aus dem Immunreagenz entfernt werden. Wie dem Fachmann
vertraut ist, findet die Immunpräzipitation zwischen
$4^{\circ}$C und $42^{\circ}$C in einem pH-Milieu zwischen 5 und 9, vorzugsweise bei $25^{\circ}$C und pH 7 bis 8,6 statt.

Wie anhand von Vergleichsuntersuchungen festgestellt
wurde, stimmt die in der dargelegten Weise durchgeführte Analytik der direkten Bestimmung z.B. von
Cholesterin in den LDL oder HDL aus Serum oder Plasma
menschlicher Herkunft mit normalen bzw. nur leicht
erhöhten Lipidkonzentrationen hervorragend mit den
Messungen mit Hilfe der herkömmlichen Verfahren überein. Bei höheren Lipidkonzentrationen, insbesondere
bei höheren Triglyceridkonzentrationen ist diese
Trennung der Lipoproteinfraktionen in vereinzelten
Seren oder Plasmen unvollständig, wenn die Fraktionierung durch eine Immunpräzipitation mit einem Reagenz, das nicht an eine Festphase gekoppelt ist, ausgeführt wird. Es hat sich daher als vorteilhaft erwiesen, wenn die Präzipitation in Gegenwart von einem
oder mehreren Enzymen, die die kolloid-chemische Stabilität der Lipoproteinpartikel herabsetzen, ausgeführt
wird. Durch die gleichzeitige enzymatische Behandlung
können noch gelöste Immunkomplexe ebenfalls präzipitiert
werden.

5

Die Verwendung verschiedener Enzyme hat sich hierfür
als vorteilhaft erwiesen:

EC 3.2.1.18  Neuraminidase
EC 3.4.22.2  Papain
EC 3.4.21.4  Pronase
EC 3.1.4.12  Sphingomyelinase
EC 3.1.4.3.  Phospholipase C
EC 3.1.1.13  Cholesterinesterase
EC 3.1.1.3   Triglyceridlipase
EC 3.1.1.1.  Carboxylesterase

Die notwendigen Mengen der Enzyme für eine Bestimmung sind durch die Art der Immunpräzipitation und
durch Spezifität und Aktivität des jeweiligen Enzyms
bestimmt.

Ein weiterer Gegenstand der  Erfindung ist ein Reagenz
zur selektiven Entfernung von störenden Bestandteilen
in einer wässrigen Lösung durch eine Immunreaktion zum
Zwecke des nachfolgenden direkten Nachweises eines
Analyts in der Flüssigkeit. Das Reagenz ist dadurch
gekennzeichnet, daß es Antikörper gegen die den Nachweis
störenden Bestandteile enthält. Den nachzuweisenden Analyt enthält es hingegen nicht oder nur in Mengen,
deren  Beitrag für den quantitativen Nachweis der Konzentrationen des Analyts in einer Probenlösung rechnerisch berücksichtigt werden kann. Die Spezifität der
Antikörper richtet sich, wie sich schon aus der Darlegung zum Verfahren ergibt, gegen die jeweiligen störenden und damit zu entfernenden Bestandteile in der
Probenlösung. Die Antikörper können hierbei in dem
Reagenz in Lösung vorliegen oder auch an eine Festphase

6

gebunden sein.

Im Falle der Entfernung der störenden Bestandteile
einer Probenlösung durch Immunpräzipitation enthält das Reagenz vorzugsweise ein oder mehrere Enzyme, die die Oberflächeneigenschaften der störenden Bestandteile in dem Sinne verändern, daß eine
Immunpräzipitation begünstigt wird. Die verwendeten
Enzyme entsprechen denen, die in der Darlegung der
Ausführung des Verfahrens vermerkt sind. Im Falle
der Aufbereitung der Probe durch Immunadsorption
haben sich die dem Fachmann vertrauten Festphasen
zur Fixation der Antikörper (Zellulose, Sepharose,
Dextran, Polyakrylamid, controlled pore glass etc)
als anwendbar erwiesen.

Die folgenden Beispiele sollen die Vorteile des
Verfahrens verdeutlichen:

Beispiel 1: Jeweils 5 ul von triglyceridarmen Humanseren wurden mit jeweils 50 ul eines delipidierten
Serums vom Kaninchen mit Antikörpern gegen Human-
Apolipoprotein A . . durchmischt. Nach einer Stunde Aufbewahrung bei Raumtemperatur wurde die Mischung in einer Laborzentrifuge zentrifugiert und
der Cholesteringehalt in der überständigen Lösung
mit einer gebräuchlichen enzymatischen Meßmethode
ermittelt. Aus den gleichen Seren wurde der Gehalt
an LDL-Cholesterin mit Hilfe der herkömmlichen
Technik der Ultrazentrifugation und Polyanionenpräzipitation sowie mit Hilfe der Methode nach Friedewald gemessen. Die Ergebnisse der Messungen von
verschiedenen Serumproben sind in der Tabelle 1 zum
Vergleich aufgeführt.

Beispiel 2: Jeweils 50 ul Serum wurden mit jeweils
1 ml 0,15 molarer NaCl verdünnt. In dieser Lösung
wurde eine an Sepharose gekoppelte Immunglobulinfraktion, die Antikörper gegen Human-Apo-A enthielt,
suspendiert, die Lösung wurde 15 min. leicht geschüttelt, die Festphase abzentrifugiert und der Cholesteringehalt in der überständigen Lösung mit einem
chemischen Verfahren (Technicon Bulletin N 24a, Tarrytown 1972) gemessen. Die Ergebnisse sind mit der Bestimmung von LDL-Cholesterin in Tabelle 1 verglichen.

Beispiel 3:  500 Mikroliter der in Beispiel 2 beschriebenen Serumverdünnungen wurden über eine kleine
Säule mit den gleichen an Sepharose gebundenen Immunglobulinen chromatographiert und der Cholesteringehalt im Eluat gemessen. Zur Ermittlung der durch die
Chromatographie bedingten Verdünnung wurde der Gehalt
an Lithium Chlorid,das der Lösung vor der Chromatographie zugesetzt worden war, vor und nach Eluation gemessen. Die Ergebnisse dieser Untersuchungen sind
in Tab. 1 ergänzt.

Beispiel 4:  66 verschiedene Seren mit einem normalen
Gehalt oder pathologisch erhöhten Gehalt an Cholesterin
und/oder Triglycerid  wurden mit einem delipidierten
Antiserum, das Antikörper gegen menschliche Apolipoproteine A und C und Neuraminidase (10 mU) enthielt,
wie in Beispiel 1 beschrieben untersucht. Die Ergebnisse wurden mit der LDL-Cholesterinbestimmung nach der
Methode von Friedewald verglichen. Der Korrelationsfaktor zwischen beiden Bestimmungsmethoden betrug:

r: 0,97 bei einer Regression von
y= 0,89 x + 0,078 .

(y: Cholesterinkonzentration (g/l) nach Immunpräzipitation; x: Cholesterinkonzentration (g/l) nach Berechnung aus der Friedewaldformel).

Beispiel 5:   In gleicher Weise wie in Beispiel 1
beschrieben wurden Humanseren mit einem delipidierten Antiserum gegen Human-Apo B in Gegenwart von
Triglyceridlipase ( 500 mU ) und Carboxylesterase
( 5 U ) vermischt. Nach einer Stunde wurde die
Mischung zentrifugiert und im Überstand der Gehalt
an Cholesterin gemessen. Die Ergebnisse sind in
Tabelle 2 mit Referenzverfahren der HDL-Cholesterinbestimmung nach Polyanionenpräzipitation aus dem
Vollserum verglichen.

Die Ergebnisse der angeführten Beispiele veranschaulichen, daß mit Hilfe der verschiedenen Variationen
des entwickelten Verfahrens präzise direkte Messungen
von Cholesterin in einer Lipoproteinklasse des menschlichen Serums, die nicht mit einem lipidfreien Reagenz
mit bestimmten immunologischen Eigenschaften reagiert,
aus minimalen Probenvolumina ohne aufwendige labortechnische Hilfsmittel möglich sind. Selbstverständlich gelingt auch die Bestimmung des Gehaltes von
anderen Lipiden, z.B. Triglycerid oder Phospholipid,
in der gleichen Lipoproteinfraktion, da auch diese
Lipide in dem Reagenz nicht in störender Weise enthalten sind. Das Verfahren bietet somit beträchtliche
Vorteile, z.B. für die klinisch-chemische Differentialdiagnostik von Fettstoffwechselstörungen; insbesondere
ist die neubeschriebene Analytik wesentlich einfacher
und kostengünstiger als die herkömmlicherweise eingesetzten Verfahren.

Das Prinzip des am Beispiel der Analytik von Serumlipoproteinen erläuterten Verfahrens kann in analoger Weise durch Verwendung eines Reagenz mit geeigneten spezifischen immunologischen Merkmalen zur
Untersuchung eines Analyts in anderen Flüssigkeiten
biologischer Herkunft (z.B.Zellkulturmedium,Urin,
Liquor etc.),der an Biopolymere mit unterschiedlichen
immunogenen Eigenschaften gebunden ist,problemlos
zu analytischen oder präparativen Zwecken angewandt
werden.

Tabelle 1    Bestimmung von "LDL"-Cholesterin

| I | II | III | IV | V |
|---|----|-----|----|----|
| 5,09 g/L | 5,45 g/L | 5,21 g/L | 5,54 g/L | 5,34 g/L |
| 1,36 " | 1,20 " | 1,19 " | 1,15 " | 1,09 " |
| 1,37 " | 1,53 " | 1,32 " | 1,24 " | 1,32 " |
| 1,48 " | 1,47 " | 1,45 " | 1,42 " | 1,35 " |
| 1,24 " | 1,30 " | 1,12 " | 1,05 " | 1,05 " |
| 1,11 " | 1,23 " | 1,19 " | 1,40 " | 1,15 " |
| 1,18 " | 1,10 " | 1,12 " | 1,22 " | 1,24 " |
| 1,45 " | 1,40 " | 1,32 " | 1,25 " | 1,35 " |
| 2,14 " | 2,22 " | 1,98 " | 2,30 " | 1,95 " |
| 1,01 " | 1,02 " | 0,95 " | 1,10 " | 0,93 " |

I:   LDL-Cholesterin (nach Ultrazentrifugation)
II:   LDL-Cholesterin (nach Friedewald)
III: Cholesterin (nach Verfahren Beispiel 1)
IV:  Cholesterin (nach Verfahren Beispiel 2)
V:   Cholesterin (nach Verfahren Beispiel 3)

Tabelle 2   Bestimmung von  HDL -Cholesterin

| Serum | I | | II | |
|-------|-----------|-----|-----------|-----|
| 1 | 0,400 | g/L | 0,430 | g/L |
|   | 0,220 | " | 0,230 | " |
|   | 0,420 | " | 0,460 | " |
|   | 0,350 | " | 0,330 | " |
|   | 0,760 | " | 0,820 | " |
|   | 0,320 | " | 0,330 | " |
|   | 0,570 | " | 0,630 | " |
|   | 0,280 | " | 0,300 | " |
|   | 0,640 | " | 0,730 | " |
|   | 0,580 | " | 0,650 | " |

I  Cholesterinkonzentrationen im Serum nach Fällung
   mit Phosphorwolframsäure/$MgCl_2$

II Cholesterinkonzentrationen im Serum nach Immunpräzipitation und Gammaglobulin gegen Apolipoprotein B in Gegenwart von
   Triglyceridlipase (EC  3.1.1.3 ) und
   Carboxylesterase  (EC  3.1.1.1 )

Verfahren und Reagenz zum direkten Nachweis eines Analyts
nach spezifischer Entfernung von Colloidpartikeln in
wässrigen Lösungen biologischer Herkunft


## Patentansprüche


1.  Verfahren zur direkten Bestimmung eines Analyts aus
    einer bestimmten Fraktion einer wässrigen Flüssigkeit
    biologischer Herkunft, dadurch gekennzeichnet, daß
    andere den Analyt ebenfalls enthaltende Fraktionen
    durch eine Immunreaktion entfernt werden und anschließend der direkte Nachweis des Analyts mit Hilfe
    einer chemischen oder biochemischen Meßmethode geführt
    wird.

2.  Verfahren nach Anspruch 1, dadurch gekennzeichnet, daß
    die Entfernung der den Nachweis des Analyts störenden
    Fraktionen durch eine Immunpräzipitation oder Immunadsorption mit Hilfe spezifischer Antikörper erfolgt.

3.  Verfahren nach Anspruch 2, dadurch gekennzeichnet, daß
    die zu entfernenden Fraktionen Lipoproteine sind,
    welche Apolipoprotein A oder C oder beide gemeinsam
    enthalten und die Immunreaktion mit Antikörpern gegen
    Apolipoprotein A oder C oder mit Antikörpern gegen die
    Apolipoproteine A und C erfolgt.

4. Verfahren nach Anspruch 2, dadurch gekennzeichnet, daß die zu entfernende Fraktionen Lipoproteine sind, welche Apolipoprotein B enthalten, und die Immunreaktion mit Antikörpern gegen Apolipoprotein B erfolgt.

5. Verfahren nach einem der Ansprüche 1-4, dadurch gekennzeichnet, daß außerdem ein oder mehrere Enzyme verwendet werden, die bewirken, daß die kolloidchemische Stabilität der zu entfernenden Fraktionen vermindert wird.

6. Verfahren nach Anspruch 5, dadurch gekennzeichnet, daß man als Enzym Neuraminidase, Pronase, Papain, Triglyceridlipase, Carboxylesterase, Cholesterinesterase, Sphingomyelinase, Phospholipase oder eine Mischung derselben verwendet.

7. Reagenz für den direkten Nachweis eines Analyts in einer bestimmten Fraktion einer wässrigen Flüssigkeit biologischer Herkunft, dadurch gekennzeichnet, daß es den Analyt nicht oder in Mengen enthält, bei deren Berücksichtigung der quantitative Nachweis des Analyts nicht beeinträchtigt wird.

8. Reagenz nach Anspruch 7, dadurch gekennzeichnet, daß es spezifische Antikörper enthält, die gegen die immunogenen Determinanten der den Nachweis eines Analyts in einer bestimmten Fraktion störenden und zu entfernenden Fraktionen gerichtet sind.

9. Reagenz zur Entfernung von Lipoproteinen, welche Apolipoprotein A oder C oder beide gemeinsam enthalten, aus menschlichem Plasma oder Serum nach einem der Ansprüche 7 und 8, dadurch gekennzeichnet, daß es Antikörper gegen Apolipoprotein A oder C oder Antikörper gegen die Apolipoproteine A und C enthält.

- 3 -

0129696

10. Reagenz zur Entfernung von Apolipoprotein B haltigen Lipoproteinen nach einem der Ansprüche 7 und 8, dadurch gekennzeichnet, daß es Antikörper gegen Apolipoprotein B enthält.

11. Reagenz nach einem der Ansprüche 7-10, dadurch gekennzeichnet, daß die Antikörper zur Entfernung der störenden Fraktionen gelöst oder an eine Festphase gebunden sind.

12. Reagenz nach einem der Ansprüche 7-11, dadurch gekennzeichnet, daß es zusätzlich ein Enzym oder eine Mischung von Enzymen enthält, deren Wirkung die Kolloid-Stabilität der zu entfernenden Fraktionen vermindert.

13. Reagenz nach Anspruch 12, dadurch gekennzeichnet, daß als Enzym Neuraminidase, Pronase, Triglyceridlipase, Carboxylesterase, Cholesterinesterase, Sphingomyelinase, Phospholipase oder Mischungen derselben enthält.

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | FR-A-2 381 311 (AKZO N.V.)<br><br>* Seiten 3-10 *<br><br>--- | 1,2,4, 7,8,10 ,11 | G 01 N 33/50<br>G 01 N 33/92 |
| P,X<br>Y | EP-A-0 092 801 (BOEHRINGER MANNHEIM GmbH)<br>* Seite 4, Zeile 14 - Seite 9; Ansprüche 1,3,5,7,10,11 *<br><br>--- | 1-3,7-9,11 | |
| Y | EP-A-0 008 338 (DR. C.-C. HEUCK)<br>* Seite 9, Ansprüche 1,2 *<br><br>--- | 5,6,12 ,13 | |
| Y | CHEMICAL ABSTRACTS, Band 94, Nr. 15, 13. April 1981, Seite 479, Nr. 118919a, Columbus, Ohio, US; W. STOFFEL u.a.: "Selective removal of apolipoprotein B-containing serum lipoproteins from blood plasma" & PROC. NATL. ACAD. SCI. USA 1981, 78(1), 611-15<br>* Zusammenfassung *<br><br>--- | 1,2,4, 7,8,10 ,11 | |

**RECHERCHIERTE SACHGEBIETE (Int. Cl. ³)**

G 01 N
C 12 Q

| Kategorie | Kennzeichnung des Dokuments | Betrifft |
|---|---|---|
| A | WO-A-8 002 460 (LABORATOIRES GÖELLA)<br><br>----- | |

Der vorliegende Recherchenbericht wurde für alle Patentansprüche erstellt.

| Recherchenort | Abschlußdatum der Recherche | Prüfer |
|---|---|---|
| DEN HAAG | 12-09-1984 | GRIFFITH G. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82